# EUROPÄISCHE PATENTSCHRIFT

(11) **EP 1 472 217 B1**
(45) Veröffentlichungstag und Bekanntmachung des Hinweises auf die Patenterteilung: **24.01.2007**
(21) Anmeldenummer: 02787800.8
(22) Anmeldetag: 25.11.2002
(51) Int. Cl.: C07C 303/26, C07C 309/65, C07D 233/58, C07D 295/02, H01M 6/16, H01M 10/40

(54) **VERFAHREN ZUR HERSTELLUNG VON PERFLUORALKANSULFONSÄUREESTERN UND DEREN SALZEN**
METHOD FOR PRODUCING PERFLUOROALKANE SULFONIC ACID ESTERS AND THE SALTS THEREOF
PROCEDE POUR PRODUIRE DES ESTERS D'ACIDE PERFLUOROALCANESULFONIQUE ET DES SELS DE CEUX-CI

(30) Priorität: 21.12.2001 DE 10163458
(43) Veröffentlichungstag der Anmeldung: 03.11.2004
(73) Patentinhaber: Merck Patent GmbH, 64271 Darmstadt (DE)
(72) Erfinder: SCHMIDT, Michael, 64342 Seeheim-Jugenheim (DE); IGNATYEV, Nicolai, 47058 Duisburg (DE); HEIDER, Udo, Winchester SO22-4HZ (GB); SARTORI, Peter, 86919 Utting (DE); KUCHERYNA, Andrij, 42117 Wuppertal (DE)
(86) Internationale Anmeldenummer: PCT/EP2002/013222
(87) Internationale Veröffentlichungsnummer: WO 2003/053918

(56) Entgegenhaltungen:
- WO-A-01/15258
- WO-A-90/14676
- WO-A-02/098844
- T. GRAMSTAD, ET AL.: "Perfluoroalkyl derivatives of sulphur. Part IV. Perfluoroalkanesulphonic acids" JOURNAL OF THE CHEMICAL SOCIETY, Nr. 1, 1956, Seiten 173-180, XP002211121 ROYAL SOCIETY OF CHEMISTRY, LETCHWORTH, GB ISSN: 0368-1769 in der Anmeldung erwähnt
- T.M. SU, ET AL.: "The solvolysis of highly unreactive substrates using the trifluoromethanesulphonate leaving group" JOURNAL OF THE AMERICAN CHEMICAL SOCIETY, Bd. 91, Nr. 19, 10. September 1969 (1969-09-10), Seiten 5386-5388, XP002211122 AMERICAN CHEMICAL SOCIETY, WASHINGTON, DC, US ISSN: 0002-7863 in der Anmeldung erwähnt
- Y. NITTA, ET AL.: "Reaction of carboxylic acid esters with p-toluenesulphonic acid" CHEMICAL AND PHARMACEUTICAL BULLETIN, Bd. 33, Nr. 4, April 1985 (1985-04), Seiten 1380-1386, XP002239338 PHARMACEUTICAL SOCIETY OF JAPAN, TOKYO, JP ISSN: 0009-2363
- U. ZOLLER: "The cheletropic fragmentation of hypervalent three-membered thiaheterocyclic intermediates" TETRAHEDRON, Bd. 44, Nr. 24, Dezember 1988 (1988-12), Seiten 7413-7426, XP002249658 ELSEVIER SCIENCE PUBLISHERS, AMSTERDAM, NL
- D.C.R. HOCKLESS, ET AL.: "1-Methyl-phenylphosphiranium triflate: synthesis, structure and reactivity" JOURNAL OF THE CHEMICAL SOCIETY, CHEMICAL COMMUNICATIONS, Nr. 2, 21. Januar 1995 (1995-01-21), Seiten 257-258, XP002211152 ROYAL SOCIETY OF CHEMISTRY, LETCHWORTH, GB ISSN: 0022-4936
- D. YANG, ET AL.: "Design of efficient ketone catalysts for epoxidation by using the field effect" JOURNAL OF ORGANIC CHEMISTRY, Bd. 63, Nr. 24, 29. Oktober 1998 (1998-10-29), Seiten 8952-8956, XP002211153 AMERICAN CHEMICAL SOCIETY, WASHINGTON, DC, US ISSN: 0022-3263
- J.F. KING, ET AL.: "Betylates. 3. Preparative nucleophilic substitution by way of [2]-, [3]-, and [4]betylates. Stoichiometric phase transfer and substrate-reagent ion-pair (SRIP) reactions of beylates" JOURNAL OF THE AMERICAN CHEMICAL SOCIETY, Bd. 104, Nr. 25, 15. Dezember 1982 (1982-12-15), Seiten 7108-7122, XP002211154 AMERICAN CHEMICAL SOCIETY, WASHINGTON, DC, US ISSN: 0002-7863
- S.E. DENMARK, ET AL.: "CatalytIc epoxidation of alkenes with oxone" JOURNAL OF ORGANIC CHEMISTRY, Bd. 60, Nr. 5, 1995, Seiten 1391-1407, XP000917828 AMERICAN CHEMICAL SOCIETY, WASHINGTON, DC, US ISSN: 0022-3263
- U. CHIACCHIO, ET AL.: "A general synthetic approach to 5-alkyl-2(5H)furanones via 1,3-dipolar cycloaddition" TETRAHEDRON, Nr. 21, 21. Mai 1998 (1998-05-21), Seiten 5695-5708, XP004118419 ELSEVIER SCIENCE PUBLISHERS, AMSTERDAM, NL ISSN: 0040-4039
- P. BONHÔTE, ET AL.: "Hydrophobic, highly conductive ambient-temperature molten salts" INORGANIC CHEMISTRY, Bd. 35, Nr. 5, 28. Februar 1996 (1996-02-28), Seiten 1168-1178, XP002249659 AMERICAN CHEMICAL SOCIETY, WASHINGTON, DC, US
- T. KITAZUME, ET AL.: "Preparation of fluorinated alkenes in ionic liquids" JOURNAL OF FLUORINE CHEMISTRY, Bd. 106, Nr. 2, Dezember 2000 (2000-12), Seiten 211-215, XP004219165 ELSEVIER SEQUOIA, LAUSANNE, CH ISSN: 0022-1139
- D.C.R. HOCKLESS, ET AL.: "Facile syntheses and interconversions between simple phosphiranium and phosphirenium salts" JOURNAL OF ORGANOMETALLIC CHEMISTRY, Bd. 529, Nr. 1, 15. Februar 1997 (1997-02-15), Seiten 189-196, XP004061298 ELSEVIER-SEQUOIA, LAUSANNE,CH ISSN: 0022-328X
- H. HEYDT, ET AL.: "Organophosphorus compounds; 122. Alkylation of 1H-phosphirenes with triflates - synthesis of lambda5,sigma4-1H-phosphirenium cations" SYNTHESIS, Nr. 2, Februar 1998 (1998-02), Seiten 175-180, XP002249729 GEORG THIEME VERLAG, STUTTGART, DE
- M.L. DI VONA, ET AL.: "Ring-opening reactions. Part 4. The role of strain and stereochemical effects on the elimination and substitution reactions of small rings; the reactivity of 1,1-dimethylaziridinium systems" JOURNAL OF THE CHEMICAL SOCIETY, PERKIN TRANSACTIONS 2, Nr. 12, Dezember 1985 (1985-12), Seiten 1943-1946, XP002249730 ROYAL SOCIETY OF CHEMISTRY, LETCHWORTH, GB
- K. ROUSSEAU, ET AL.: "Tetraalkylammonium trifluoromethanesulphonates as supporting electrolytes" JOURNAL OF ORGANIC CHEMISTRY, Bd. 37, Nr. 27, 1. Dezember 1972 (1972-12-01), Seiten 3968-3971, XP002239601 AMERICAN CHEMICAL SOCIETY, WASHINGTON, DC, US ISSN: 0022-3263
- PATENT ABSTRACTS OF JAPAN Bd. 016, Nr. 582 (E-1300), 22. Dezember 1992 (1992-12-22) & JP 04 233210 A (MURATA MANUFACTURING), 21. August 1992 (1992-08-21)
- B.L. BOOTH, ET AL.: "Alkyltrifluoromethanesulphonates as alkylating reagents for aromatic compounds" JOURNAL OF THE CHEMICAL SOCIETY, PERKIN TRANSACTIONS 1, Nr. 12, Dezember 1980 (1980-12), Seiten 2887-2893, XP002249731 ROYAL SOCIETY OF CHEMISTRY, LETCHWORTH, GB
- R.E. BATES, ET AL.: "Alkylation of enolates with triflates" JOURNAL OF ORGANIC CHEMISTRY, Bd. 58, Nr. 16, 30. Juli 1993 (1993-07-30), Seiten 4469-4470, XP002249732 AMERICAN CHEMICAL SOCIETY, WASHINGTON, DC, US
- F. MARCUZZI, ET AL.: "Vinyl cations in organic synthesis. A new route to disubstituted alkynes" JOURNAL OF ORGANIC CHEMISTRY, Bd. 47, Nr. 23, 5. November 1982 (1982-11-05), Seiten 4577-4579, XP002249733 AMERICAN CHEMICAL SOCIETY, WASHINGTON, DC, US
- C.D. BEARD, ET AL.: "Synthesis of some novel trifluoromethanesulphonates and their reactions with alcohols" JOURNAL OF ORGANIC CHEMISTRY, Bd. 38, Nr. 21, 19. Oktober 1973 (1973-10-19), Seiten 3673-3677, XP002249734 AMERICAN CHEMICAL SOCIETY, WASHINGTON, DC, US
- M. OBA, ET AL.: "Reaction of N-trimethylsilylmethyliminodithiocarbonate with carbonyl compounds: synthetic equivalent of alkylthionitrile ylide" HETEROCYCLES, Bd. 45, Nr. 10, 1. Oktober 1997 (1997-10-01), Seiten 1913-1919, XP002249735 ELSEVIER SCIENCE PUBLISHERS, AMSTERDAM, NL
- E. HONDA, ET AL.: "Novel ring transformation of dihydroselenines to selenabicyclo[3.1.0]hexenes" HETEROCYCLES, Bd. 55, Nr. 3, 1. März 2001 (2001-03-01), Seiten 465-468, XP002249736 ELSEVIER SCIENCE PUBLISHERS, AMSTERDAM, NL

## Beschreibung

Die vorliegende Erfindung betrifft ein Verfahren zur Herstellung von Verbindungen mit Perfluoralkansulfonsäureresten, insbesondere die Herstellung von Perfluoralkansulfonsäureestern und deren weitere Umsetzung zu Salzen.

Die Verbreitung von tragbaren elektronischen Geräten, wie z.B. Laptop- und Palmtop-Computem, Mobiltelefonen, oder Videokameras und damit auch der Bedarf nach leichten und leistungsfähigen Batterien hat in den letzten Jahren weltweit dramatisch zugenommen. Angesichts dieses sprunghaft gestiegenen Bedarfs nach Batterien und den damit verbundenen ökologischen Problemen kommt der Entwicklung von wiederaufladbaren Batterien mit einer langen Lebensdauer eine stetig wachsende Bedeutung zu.

Lithium-lonen-Batterien und Doppelschichtkondensatoren mit sehr hohen Kapazitäten (sogenannte Super- oder Ultracapacitors) stellen den derzeitigen Stand der Technik dar. In beiden Systemen werden derzeit mit LiPF₆ bzw. N(C₂H₅)₄BF₄ hydrolyseempfindliche und thermisch instabile Substanzen als Leitsalz verwendet. Im Kontakt mit feuchter Luft bzw. mit Restwasser aus den Lösungsmitteln kann schnell HF entstehen. Neben den toxischen Eigenschaften wirkt HF sehr negativ auf das Zyklenverhalten und somit auf die Performance der elektrochemischen Zellen.

Als Alternative wurden Imide, wie das Bis(trifluormethylsulfonyl)imid oder das Bis(pentafluorethylsulfonyl)imid oder Methanide, wie das Tris(trifluormethylsulfonyl)methanid und deren Derivate vorgestellt. Aber auch quaternäre Ammonium- und Phosphoniumsalze mit Perfluoralkansulfonat-Anionen wurden als Leitsalze für galvanische Zellen entwickelt. Die Synthese dieser Salze ist jedoch relativ aufwendig, da ein Zwischenprodukt, der Trifluormethansulfonsäuremethylester (Methyltriflat), schwer darzustellen ist.

Es gibt verschiedene Synthesewege für das Methyltriflat (Gramstad, J. Chem. Soc., 1956, 173-180 oder Beard, J. Org. Chem., 1973 (21), 3673-3677). Alle beschriebenen Synthesewege eignen sich jedoch nicht für eine Übertragung in einen großen Maßstab, da sie entweder sehr toxische Ausgangsmaterialien, wie z.B. Dimethylsulfat, verwenden, die Ausbeuten sehr niedrig sind, das Reaktionsprodukt aufgereinigt werden muss, oder gefährliche Neben- bzw. Abfallprodukte anfallen, wie z.B. Schwefelsäure die mit Dimethylsulfat verunreinigt ist.

Aufgabe der vorliegenden Erfindung ist es daher, die Nachteile des Standes der Technik zu überwinden und ein einfaches und wirtschaftlich effektives Verfahren für die Synthese von Perfluoralkansulfonsäurealkylestern und daraus herstellbare Leitsalze zur Verfügung zu stellen.

Gelöst wird diese Aufgabe durch die Verfahren gemäß Anspruch 1 und Anspruch 9. Spezielle Verfahrensmerkmale sind in den Unteransprüchen 2 bis 8 beschrieben.

Die Erfindung zeichnet sich dadurch aus, dass Perfluoralkansulfonsäure direkt mit Dialkylcarbonat zu Perfluoralkansulfonsäurealkylester umgesetzt wird. Beispielsweise kann Trifluormethansulfonsäure direkt mit Dimethylcarbonat umgesetzt werden. Die Bildung des Methyltriflats erfolgt jedoch nur in geringen Ausbeuten (vgl. Beispiel 1).

Bessere Ausbeuten liefert die bevorzugt durchgeführte Umsetzung von Perfluoralkansulfonsäure mit Dialkylcarbonat in Gegenwart eines wasser- oder alkoholverbrauchenden Reagenz wie z.B. eines Carbonsäurederivates, dessen organischer Rest gegenüber Perfluoralkansulfonsäure stabil ist, z.B.

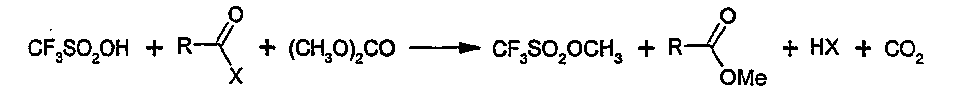

Ein Carbonsäurederivat im Sinne der vorliegenden Erfindung ist eine Verbindung, bei der die Hydroxigruppe einer Carbonsäure durch eine andere funktionelle Gruppe, beispielsweise ein Halogenid, einen Carbonsäurerest oder einen Sulfonsäurerest, ersetzt ist. Erfindungsgemäß sind grundsätzlich alle Carbonsäurederivate einsetzbar, sofern deren Alkyl- oder Arylreste - auch die protonenhaltigen - gegen Perfluoralkansulfonsäure stabil sind.

Überraschenderweise erfolgt die Alkylierung der Mischung aus Perfluoralkansulfonsäure und Carbonsäurederivat leicht und führt zu guten

Ausbeuten an alkylierter Perfluoralkansulfonsäure und Carbonsäureester. Beide Verbindungen kann der Fachmann nach konventionellen Methoden, in der Regel durch fraktionierte Destillation, leicht isolieren.

In einer bevorzugten Ausführungsform werden als Carbonsäurederivate für das erfindungsgemäße Verfahren Carbonsäurehalogenide, insbesondere Chloride, Carbonsäureanhydride oder gemischte Carbonsäure-Sulfonsäureanhydride eingesetzt. Die Verwendung dieser Edukte führt in relativ kurzen Reaktionszeiten zu guten Ausbeuten der Ester.

Besonders bevorzugt ist das Carbonsäurechlorid ausgewählt aus der Gruppe: Benzoylchlorid, p-Nitrobenzoylchlorid, 2,6-Difluorbenzoylchlorid, Pentafluorbenzoylchlorid, 2-Chlorbenzoylchlorid, 3-Chlorbenzoylchlorid, 4-Chlorbenzoylchlorid, 2-Brombenzoylchlorid, 3-Brombenzoylchlorid, 4-Brombenzoylchlorid, 2,3-Dichlorbenzoylchlorid, 2,4-Dichlorbenzoylchlorid, 2,6-Dichlorbenzoylchlorid, 3,4-Dichlorbenzoylchlorid, 3,5-Dichlorbenzoylchlorid, Trichloracetylchlorid.

Als Carbonsäureanhydrid ist besonders bevorzugt Benzoesäureanhydrid, 2,2'-Dichlorbenzoesäureanhydrid, 3,3'-Dichlorbenzoesäureanhydrid, 4,4'-Dichlorbenzoesäureanhydrid, 2,2',3,3'-Tetrachlorbenzoesäureanhydrid, 2,2',4,4'-Tetrachlorbenzoesäureanhydrid, 2,2',6,6'-Tetrachlorbenzoesäureanhydrid, 3,3',4,4'-Tetrachlorbenzoesäureanhydrid, 3,3',5,5'-Tetrachlorbenzoesäureanhydrid, 2-Brombenzoesäureanhydrid, 3-Brombenzoesäureanhydrid, 4-Brombenzoesäureanhydrid, 2,2',6,6'-Tetrafluorbenzoesäureanhydrid.

Als erfindungsgemäß verwendetes Dialkylcarbonat kann der Fachmann grundsätzlich jedes bekannte Dialkylcarbonat einsetzen. Bevorzugt ist es jedoch ausgewählt aus der Gruppe Dimethylcarbonat, Diethylcarbonat, Dipropylcarbonat, Dibutylcarbonat, Methylethylcarbonat oder eine Mischung dieser Dialkylcarbonate.

Vorzugsweise wird das erfindungsgemäße Verfahren bei Temperaturen zwischen Raumtemperatur und 150°C, insbesondere zwischen 50 und 110°C, ganz besonders bevorzugt zwischen 70 und 100°C, durchgeführt. Die bevorzugte Reaktionszeit liegt zwischen 1 und 10 Stunden, insbesondere zwischen 2 und 5 Stunden.

Die erfindungsgemäß hergestellten Perfluoralkansulfonsäureester können anschließend zu den entsprechenden Perfluoralkansulfonsäuresalzen durch Reaktion mit

XR¹R²R³,

weiter umgesetzt werden, wobei
- X: P oder N
- R¹, R², R³: gleich oder verschieden sind, gegebenenfalls durch eine Einfach- oder Doppelbindung direkt miteinander verbunden sind und jeweils einzeln oder gemeinsam die Bedeutung
- eines Wasserstoffs,
- eines Alkylrestes mit 1 bis 16 C-Atomen, der teilweise oder vollständig mit weiteren Gruppen substituiert sein kann, vorzugsweise mit F, Cl, N(CₙF₍₂ₙ₊₁₋ₓ₎Hₓ)₂, O(CₙF₍₂ₙ₊₁₋ₓ)Hₓ), SO₂(CₙF₍₂ₙ₊₁₋ₓ₎Hₓ), CₙF₍₂ₙ₊₁₋ₓ₎Hₓ mit 1≤n≤6 und 0≤x≤2n+1, ggf. substituiertem Arylrest oder ggf. substituiertem aromatischem heterocyclischem Rest,
- eines Alkylarylrestes, dessen Alkylengruppe 1 bis 16 C-Atome besitzt und der teilweise mit weiteren Gruppen, vorzugsweise mit F, Cl, Br, NO₂, CN, Alkyl, Aryl oder heterocyclisches Aryl, substituiert sein kann,
- eines Arylrestes, der teilweise mit weiteren Gruppen, vorzugsweise mit F, Cl, Br, NO₂, CN, Alkyl, Aryl oder heterocyclisches Aryl, substituiert sein kann, oder
- eines aromatischen heterocyclischen Restes, der teilweise mit weiteren Gruppen, vorzugsweise mit F, Cl, Br, NO₂, CN, Alkyl, Aryl oder heterocyclisches Aryl, substituiert sein kann,
besitzen,
wobei ein, zwei oder drei CH₂-Gruppen eines Alkylrestes durch gleiche oder verschiedene Heteroatome, vorzugsweise O, NH oder N(Alkyl) mit 1 bis 6 C-Atomen, ersetzt sein können
und wobei nicht alle drei R gleichzeitig perfluoriert oder perchloriert sein können.

Nach der Umsetzung fällt das gebildete Perfluoralkansulfonsäuresalz aus oder kann nach konventionellen Methoden isoliert werden. Der nicht umgesetzte Perfluoralkansulfonsäurealkylester muss lediglich abdestilliert werden.

Bevorzugt wird bei dieser nachfolgenden Umsetzung mit dem Ester eine Verbindung XR¹R²R³ eingesetzt, die ausgewählt ist aus der Gruppe:
X(C₂H₅)₃, X(C₃H₇)₃, X(C₄H₉)₃, 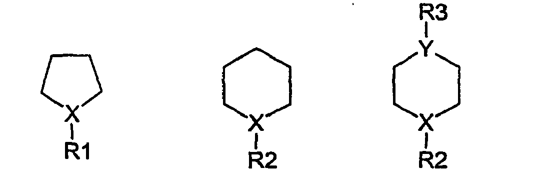 wobei
- X, Y: P oder N
- R¹, R², R³: H, Alkyl, vorzugsweise mit 1 bis 16 C-Atomen, Alkylaryl, Aryl oder heterocyclisches Aryl
bedeuten und
wobei ein, zwei oder drei CH₂-Gruppen des Rings und/oder des Alkylrests durch gleiche oder verschiedene Heteroatome, vorzugsweise O, NH oder N(Alkyl) mit 1 bis 6 C-Atomen, ersetzt sein können und
wobei der Ring und/oder der Alkylrest teilweise mit weiteren Gruppen, vorzugsweise mit F, Cl, N(CₙF₍₂ₙ₊₁₋ₓ)Hₓ)₂, O(CₙF₍₂ₙ₊₁₋ₓ₎Hₓ),
SO₂(CₙF₍₂ₙ₊₁₋ₓ₎Hₓ), CₙF₍₂ₙ₊₁₋ₓ₎Hₓ mit 1≤n≤6 und 0≤x≤2n+1, Alkylaryl, Aryl, heterocyclisches Aryl oder heterocyclisches Alkylaryl, substituiert sein kann.

Weiterhin bevorzugt wird ein erfindungsgemäß erhaltener Perfluoralkansulfonsäureester mit einer Verbindung XR¹R²R³ ausgewählt aus der folgenden Gruppe zu einem Salz umgesetzt: 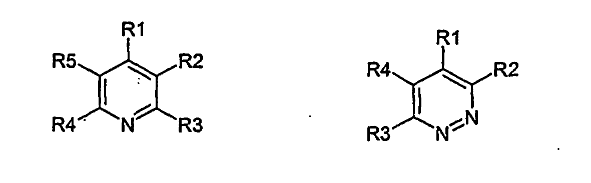 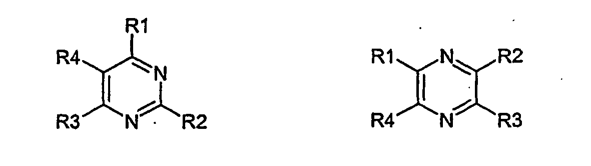 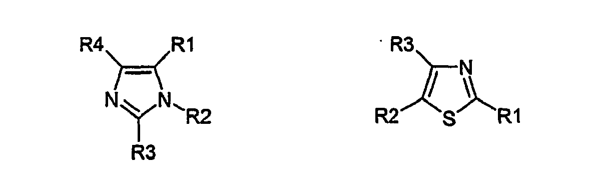 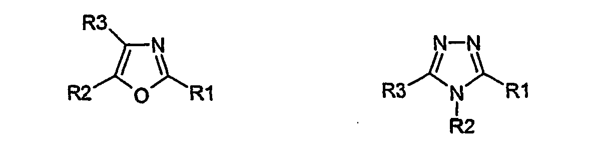 wobei R¹ bis R⁴ gleich oder verschieden sind, gegebenenfalls durch eine Einfach- oder Doppelbildung direkt miteinander verbunden sind und jeweils einzeln oder gemeinsam die Bedeutung
- eines Wasserstoffs,
- eines Halogens, vorzugsweise Fluor,
   mit der Maßgabe, dass keine N-Halogen-Bindung vorliegt,
- eines Alkylrests mit 1 bis 8 C-Atomen, der teilweise oder vollständig durch weitere Gruppen, vorzugsweise F, Cl, N(CₙF₍₂ₙ₊₁₋ₓ₎Hₓ)₂, O(CₙF₍₂ₙ₊₁₋ₓ₎Hₓ), SO₂(CₙF₍₂ₙ₊₁₋ₓ₎Hₓ), CₙF₍₂ₙ₊₁₋ₓ)Hₓ mit 1≤n≤6 und 0≤x≤2n+1, Alkylaryl, Aryl oder heterocyclisches Aryl, substituiert sein kann,
- eines Arylrestes
- eines Alkylarylrestes
- eines aromatischen heterocyclischen Restes
- eines heterocyclisches Alkylarylrestes
besitzen.

Die erfindungsgemäß hergestellten Verbindungen mit Perfluoralkansulfonsäureresten, d.h. die Perfluoralkansulfonsäureester sowie insbesondere deren Salze, können in Elektrolyten, galvanischen Zellen, primären und sekundären Batterien, Kondensatoren und/oder Super- bzw. Ultrakondensatoren, beispielsweise als Lösungsmittel oder Leitsalze, eingesetzt werden. Dabei können die Salze sowohl in reiner Form als auch in Form ihrer Mischungen als Leitsalze eingesetzt werden. Es ist auch möglich, die Salze gemeinsam mit weiteren, dem Fachmann bekannten Salzen als Leitsalz zu verwenden. Daneben sind die Perfluoralkansulfonsäureester starke Alkylierungsmittel und sind zur Alkylierung organischer Verbindungen, beispielsweise bei der Herstellung von Arzneimitteln und Pflanzenschutzmitteln, geeignet.

Die Verbindungen mit Perfluoralkansulfonsäureresten, insbesondere die Salze, können in flüssigen, gelartigen, polymeren oder festen Elektrolyten verwendet werden. Hierzu können Gemische enthaltend die Leitsalze sowie geeignete Polymeren und/oder geeignete Lösungsmittel eingesetzt werden. Ein Gemisch im Sinne der vorliegenden Erfindung umfasst reine Mischungen der Komponenten, Mischungen, in denen das oder die Salze in einem Polymeren oder Gel eingeschlossen sind sowie Mischungen, in denen zwischen dem oder den Salzen und einem Polymeren oder Gel chemische und/oder physikalische Bindungen bestehen. Im Fall eines gelartigen Elektrolyten enthält das Gemisch vorzugsweise neben dem oder den Salzen und dem Polymer ein geeignetes Lösungsmittel.

Als Lösungsmittel für flüssige oder gelartige Elektrolyte werden besonders bevorzugt aprotische Lösungsmittel oder deren Gemische eingesetzt, die zur Anwendung in einer primären oder sekundären Batterie, einem Kondensator, einem Superkondensator oder einer galvanischen Zelle geeignet sind, zum Beispiel Carbonate, Ester, Ether, Sulfolane oder Nitrile wie beispielsweise Dimethylcarbonat, Diethylcarbonat, Butylencarbonat, Propylencarbonat, Ethylencarbonat, Ethylmethylcarbonat, Methylpropylcarbonat, 1,2-Dimethoxyethan, 1,2-Diethoxyethan, Methylacetat, γ-Butyrolacton, Ethylacetat, Methylpropionat, Ethylpropionat, Methylbutyrat, Ethylbutyrat, Dimethylsulfoxid, Dioxolan, Sulfolan, Acetonitril, Acrylnitril, Tetrahydrofuran, 2-Methyltetrahydrofuran oder deren Gemische.

Als Polymere für polymere oder gelartige Elektrolyte werden vorzugsweise Homopolymere oder Copolymere von Acrylnitril, Vinylidendifluorid, Methyl(meth)acrylat, Tetrahydrofuran, Ethylenoxid, Siloxan, Phosphazen oder eine Mischung aus wenigstens zwei der vorstehend genannten Homopolymeren und/oder Copolymeren eingesetzt, wobei die Polymere zumindest teilweise vernetzt sein können.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

Alle NMR-Spektren wurden auf einem Bruker Spektrometer WP 80 SY gemessen (¹H: 80,1 MHz, ¹⁹F: 75,5 MHz).

### Beispiel 1

In einem Rundhalskolben mit Rückflusskühlerwerden 19,4 g (0,129 mol) Trifluormethansulfonsäure vorgelegt. Unter ständigem Rühren und Kühlung mit einem Eisbad werden 5,81 g (0,0646 mol) Dimethylcarbonat zugegeben. Anschließend wird die Reaktionsmischung 3 Stunden mit einem Ölbad auf 90°C (Temperatur im Ölbad) erwärmt, bis die Gasentwicklung aufhört. Nach dem Abkühlen auf Raumtemperatur wird die Reaktionsmischung bei Atmosphärendruck destilliert. Es werden 12.3 g einer transparenten, farblosen Flüssigkeit isoliert (Siedebereich 100-102°C). Dieses Gemisch enthält 96,3% Trifluormethansulfonsäuremethylester (Methyltriflat) und 3,7% Dimethylcarbonat. Die Ausbeute an Methyltriflat beträgt 55,8%.

NMR ¹⁹F, ppm (Lösungsmittel: CDCl₃, interner Standard: CCl₃F): -74,86 s (CF₃) NMR ¹H, ppm (Lösungsmittel: CDCl₃, interner Standard: TMS): 4,21 q; J⁵_{H,F} = 0,7 Hz
¹⁹F- und ¹H-NMR-Daten entsprechen den Literaturdaten des Methyltriflats (Encyclopedia of Reagents for Organic Synthesis, Editor in Chief Leo A. Paquette, Vol. 5, John Wiley and Sons Ltd., 1995, 3618; J. Org. Chem., Vol. 38, No. 21, 1973, 3673-3677)

### Beispiel 2

In einem Rundhalskolben mit Rückflusskühler werden 76,36 g (0,509 mol) Trifluormethansulfonsäure vorgelegt. Unter ständigem Rühren werden 71,60 g (0,509 mol) Benzoylchlorid innerhalb von 2 Minuten zugegeben. Dabei erwärmt sich die Mischung und eine Gasentwicklung wird beobachtet. Ohne die Reaktionsmischung abzukühlen werden 45,81 g (0,509 mol) Dimethylcarbonat zugegeben und anschließend wird die Reaktionsmischung 10 Stunden mit einem Ölbad auf 90°C (Temperatur im Ölbad) erwärmt. Nach dem Abkühlen auf Raumtemperatur wird die Reaktionsmischung bei Atmosphärendruck destilliert. Es werden 75,05 g (89,9%) Trifluormethansulfonsäuremethylester (Methyltriflat) als transparente, farblose Flüssigkeit isoliert (Siedebereich 98-99°C).

¹⁹F- und ¹H-NMR-Daten des Methyltriflats entsprechen den Literaturdaten und den in Beispiel 1 angegebenen Daten.

Der Destillationsrückstand wird unter Rühren nochmals mit 49,15 g (0,328 mol) Trifluormethansulfonsäure, 46,10 g (0,328 mol) Benzoylchlorid und 29,49 g (0,328 mol) Dimethylcarbonat versetzt. Anschließend wird die Reaktionsmischung 6 Stunden mit einem Ölbad auf 90°C (Temperatur im Ölbad) erwärmt. Danach werden 52,00 g (Ausbeute: 96,8%) reines Methyltriflat durch Destillation erhalten.

Die durchschnittliche Ausbeute des Methyltriflats der beiden aufeinander folgenden Reaktionen beträgt 92,6%.

Nach der Isolierung des Methyltriflats wird die verbleibende Reaktionsmischung unter vermindertem Druck destilliert (Siedebereich: 89-91 °C bei 2,7 kPa). Es werden 94,92 g (83,4%) reines Methylbezoat erhalten.

NMR ¹H, ppm (Lösungsmittel: CD₃CN, interner Standard: TMS): 3,86 s (CH₃), 7,52 m (3H), 8,00 m (2H)

Der nach der Destillation der Flüssigkeiten verbleibende Destillationsrückstand ist Benzoesäure, die aus Ethanol-Wasser kristallisiert werden kann (Schmelzpunkt 121-122°C).

### Beispiel 3

In einem Rundhalskolben mit Rückflusskühler werden 29,77 g (0,160 mol) p-Nitrobenzoylchlorid, 15,00 g (0,167 mol) Dimethylcarbonat und 24,07 g (0,160 mol) Trifluormethansulfonsäure gemischt und unter ständigem Rühren in einem Ölbad 2 Stunden auf etwa 75°C erwärmt (Temperatur im Ölbad). Nach dem Abkühlen auf Raumtemperatur wird das Methyltriflat unter Atmosphärendruck abdestilliert: 18,57 g (Ausbeute: 70,6%) einer transparenten, farblosen Flüssigkeit werden isoliert (Siedebereich 98-99°C).

Der feste, verbleibende Destillationsrückstand besteht hauptsächlich aus p-Nitrobenzoesäuremethylester, der nach Kristallisation aus Methanol als hellgelbes Produkt erhalten wird (25,0 g, Ausbeute: 86,0%, Schmelzpunkt: 93-94°C).

### Beispiel 4

In einem Rundhalskolben mit Rückflusskühler werden 20,84 g (0,139 mol) Trifluormethansulfonsäure vorgelegt. Unter ständigem Rühren und unter Eiskühlung werden 24,69 g (0,139 mol) 2,6-Difluorbenzoylchlorid und 12,50 g (0,139 mol) Dimethylcarbonat zugegeben. Das Eisbad wird durch ein Ölbad ersetzt und die Reaktionsmischung unter Rühren 4 Stunden auf 80-110°C erwärmt (Temperatur im Ölbad). Bei etwa 70°C beginnt eine Gasentwicklung. Nach Beendigung der Reaktion, wird die Mischung auf Raumtemperatur abgekühlt und das Methyltriflat unter Atmosphärendruck abdestilliert: 20,63 g (Ausbeute: 90,6%) einer transparenten, farblosen Flüssigkeit werden isoliert (Siedebereich 98-99°C).

Die verbleibende Reaktionsmischung wird unter vermindertem Druck destilliert (Siedepunkt: 90°C bei 2,0 kPa). Es werden 21,50 g (Ausbeute: 89,4%) reiner 2,6-Difluorbezoesäuremethylester erhalten.

NMR ¹⁹F, ppm (Lösungsmittel: CD₃CN, interner Standard: CCl₃F): -111,50 t (2F), J_{H,F} = 7,0 Hz
NMR ¹H, ppm (Lösungsmittel: CD₃CN, interner Standard: TMS): 3,92 s (CH₃), 7,04 m (2H), 7,53 m (1 H)

### Beispiel 5

In einem Rundhalskolben mit Rückflusskühler werden 16,08 g (0,107 mol) Trifluormethansulfonsäure vorgelegt. Unter ständigem Rühren und unter Eiskühlung werden 24,72 g (0,107 mol) Pentafluorbenzoylchlorid und 9,65 g (0,107 mol) Dimethylcarbonat zugegeben. Das Eisbad wird durch ein Ölbad ersetzt und die Reaktionsmischung unter Rühren 4 Stunden auf 80-110°C erwärmt (Temperatur im Ölbad). Bei etwa 75°C beginnt eine Gasentwicklung. Nach Beendigung der Reaktion, wird die Mischung auf Raumtemperatur abgekühlt und das Methyltriflat unter Atmosphärendruck abdestilliert: 14,81 g (Ausbeute: 84,2%) einer transparenten, farblosen Flüssigkeit werden isoliert (Siedebereich 98-99°C).

Die verbleibende Reaktionsmischung wird unter vermindertem Druck destilliert (Siedepunkt: 72°C bei 2,0 kPa). Es werden 21,45 g (Ausbeute: 79,7%) reiner Pentafluorbezoesäuremethylester erhalten.

NMR ¹⁹F, ppm (Lösungsmittel: CD₃CN, interner Standard: CCl₃F): -139,58 dm (2F), -150,37 tt (1 F), -161,89 m (2F), J³_{F,F} = 20,0 Hz, J⁴_{F,F} = 4,4 Hz
NMR ¹H, ppm (Lösungsmittel: CD₃CN, interner Standard: TMS): 3,96 s (CH₃)

### Beispiel 6

In einem Rundhalskolben mit Rückflusskühler werden 4,23 g (0,0187 mol) Benzoesäureanhydrid und 2,53 g (0,0187 mol) Dimethylcarbonat vorgelegt. Unter ständigem Rühren und unter Eiskühlung werden 2,81 g (0,0187 mol) Trifluormethansulfonsäure zugegeben. Das Eisbad wird durch ein Ölbad ersetzt und die Reaktionsmischung unter Rühren 4 Stunden auf 90-110°C erwärmt (Temperatur im Ölbad) bis die Gasentwicklung aufhört. Nach Beendigung der Reaktion, wird die Mischung auf Raumtemperatur abgekühlt und das Methyltriflat unter Atmosphärendruck abdestilliert: 0,55 g (Ausbeute: 17,9%) einer transparenten, farblosen Flüssigkeit werden isoliert (Siedebereich 99-100°C).

Die verbleibende Reaktionsmischung wird unter vermindertem Druck destilliert (Siedebereich: 88-93°C bei 2,0 kPa). Es werden 3,96 g (Ausbeute: 77,8%) praktisch reines Methylbenzoat erhalten.

NMR ¹H, ppm (Lösungsmittel: CD₃CN, interner Standard: TMS): 3,86 s (CH₃), 7,52 m (3H), 8,00 m (2H)

### Beispiel 7

In einem Rundhalskolben mit Rückflusskühler werden 10,73 g (0,0578 mol) p-Nitrobenzoylchlorid und 6,84 g (0,0579 mol) Diethylcarbonat vorgelegt. Unter ständigem Rühren und unter Eiskühlung werden 8,68 g (0,0579 mol) Trifluormethansulfonsäure zugegeben. Das Eisbad wird durch ein Ölbad ersetzt und die Reaktionsmischung unter Rühren 5 Stunden auf 100-110°C erwärmt (Temperatur im Ölbad) bis die Gasentwicklung aufhört. Nach Beendigung der Reaktion, wird die Mischung auf Raumtemperatur abgekühlt und der Trifluormethansulfonsäureethylester (Ethyltriflat) unter Atmosphärendruck abdestilliert: 3,28 g (Ausbeute: 31,8%) einer transparenten, farblosen Flüssigkeit werden isoliert (Siedebereich 114-116°C).

NMR ¹⁹F, ppm (Lösungsmittel: CDCl₃, interner Standard CCl₃F): -75,68 s (CF₃) NMR ¹H, ppm (Lösungsmittel: CDCl₃, interner Standard TMS): 1,51 t (CH₃), 4,62 q (CH₂), J³_{H,H} = 7,0 Hz
¹⁹F- und ¹H-NMR-Daten entsprechen den Literaturdaten des Trifluormethansulfonsäureethylesters (Ethyltriflats) (Eur. Polym. J., Vol. 16, No. 9, 1980, 861-865)

### Beispiel 8

In einem Rundhalskolben mit Rückflusskühler werden 13,67 g (0,0911 mol) Trifluormethansulfonsäure bei -30°C vorgelegt. Unter ständigem Rühren werden innerhalb von 2 Minuten 12,80 g (0,0911 mol) Benzoylchlorid zugegeben, wobei die Mischung sich leicht erwärmt. Ohne Kühlung der Mischung werden dann 10,77 g (0,0912 mol) Diethylcarbonat zugegeben. Die Reaktionsmischung wird in einem Ölbad unter Rühren 4,5 Stunden auf 70-90°C erwärmt (Temperatur im Ölbad). Bei etwa 70°C Ölbadtemperatur beginnt eine Gasentwicklung. Nach Beendigung der Reaktion, wird die Mischung auf Raumtemperatur abgekühlt und der Trifluormethansulfonsäureethylester (Ethyltriflat) unter Atmosphärendruck abdestilliert: es werden 13,10 g (Ausbeute: 80,8%) einer transparenten, farblosen Flüssigkeit isoliert (Siedepunkt 115°C).

¹⁹F- und ¹H-NMR-Daten des Ethyltriflats entsprechen den Literaturdaten (vgl. auch Beispiel 7).

Die verbleibende Reaktionsmischung wird unter vermindertem Druck destilliert (Siedepunkt: 100°C bei 2,0 kPa). Es werden 9,91 g (Ausbeute: 72,5%) reiner Bezoesäureethylester erhalten.

NMR ¹H, ppm (Lösungsmittel: CD₃CN, interner Standard: TMS): 1,35 t (3H, CH₃), 4,33 q (2H, CH₂), 7,53 m (3H), 8,00 m (2H), J³_{H,H} = 7,0 Hz
¹H-NMR-Daten entsprechen den Literaturdaten von Ethylbenzoat (The Aldrich Library of NMR Spectra, Edition II, Charles J Pouchert, Volume 2, 281)

### Beispiel 9

In einem Rundhalskolben mit Rückflusskühler werden 17,74 g (0,1182 mol) Trifluormethansulfonsäure vorgelegt. Unter ständigem Rühren werden innerhalb von 2 Minuten 21,41 g (0,1177 mol) Trichloressigsäurechlorid zugegeben. Ohne Kühlung der Mischung werden dann innerhalb von 5 Minuten 10,60 g (0,1177 mol) Dimethylcarbonat zugegeben. Die Reaktionsmischung erwärmt sich leicht und wird in einem Ölbad unter Rühren 7 Stunden auf 80-100°C erwärmt (Temperatur im Ölbad) bis die Gasentwicklung aufhört. Nach dem Abkühlen auf Raumtemperatur wird die Mischung unter Atmosphärendruck destilliert: es werden 17,48 g (Ausbeute: 90,5%) Trifluormethansulfonsäuremethylester als transparente, farblose Flüssigkeit isoliert (Siedebereich 98-100°C).

¹⁹F- und ¹H-NMR-Daten entsprechen den Literaturdaten und denen der vorhergehenden Beispiele.

Die verbleibende Reaktionsmischung wird weiterdestilliert (Siedebereich: 152-153°C). Es werden 15,16 g (Ausbeute: 72,6%) Trichloressigsäuremethylester erhalten.

NMR ¹H, ppm (Lösungsmittel: CD₃CN, interner Standard: TMS): 3,98 s (CH₃)

### Beispiel 10

In einem Rundhalskolben mit Rückflusskühler werden 6,31 g (0,0768 mol) 1-Methylimidazol in 50 ml trockenen Hexan vorgelegt. Unter ständigem Rühren und Kühlen mit Hilfe eines Eisbades werden innerhalb von 20 Minuten 13,75 g (0,0772 mol) Ethyltriflat zugegeben. Nach weiteren 10 Minuten wird dann das Eisbad durch ein Ölbad ersetzt und die Reaktionsmischung wird eine Stunde unter Rückfluss gekocht (Ölbad-Temperatur 70-75°C). Nach der Abdestillation des Hexans wird die verbleibende Reaktionsmischung 5 Stunden bei 80-90°C im Vakuum bei 30-100 Pa gehalten: es werden 19,80 g (Ausbeute: 99,1%) 1-Methyl-3-ethylimidazolium-trifluormethansulfonat als transparente, farblose Flüssigkeit erhalten.

NMR ¹⁹F, ppm (Lösungsmittel: CD₃CN, interner Standard CCl₃F): -78,05 s (CF₃SO₃⁻)
NMR ¹H, ppm (Lösungsmittel: CD₃CN, interner Standard TMS): 1,48 t (CH₃), 3,89 s (CH₃), 4,23 q (CH₂), 7,47 dd (1 H), 7,54 dd (1 H), 8,74 br.s. (1H), J³_{H,H} = 7,3 Hz, J_{H,H} = 1,8 Hz

### Beispiel 11

In einem Rundhalskolben mit Rückflusskühlerwerden 141,13 g (1,657 mol) 1-Methylpyrrolidin in 800 ml trockenen Hexan vorgelegt. Unter ständigem Rühren und Kühlen mit Hilfe eines Eisbades werden innerhalb von 45 Minuten 272 g (1,657 mol) Methyltriflat zugegeben. Dann wird das Eisbad durch ein Ölbad ersetzt und die Reaktionsmischung wird 15 min unter Rückfluss gekocht (Ölbad-Temperatur 70-75°C). Nach dem Abkühlen auf Raumtemperatur wird der weiße Niederschlag abfiltriert, zweimal mit 100 ml Hexan gewaschen und bei 110°C im Vakuum bei 30-100 Pa drei Stunden getrocknet: es werden 409 g (Ausbeute: 99,1%) 1,1-Dimethylpyrrolidinium-trifluormethansulfonat als weißer Feststoff erhalten.

NMR ¹⁹F, ppm (Lösungsmittel: CD₃CN, interner Standard CCl₃F): -78,00 s (CF₃SO₃⁻)
NMR ¹H, ppm (Lösungsmittel: CD₃CN, interner Standard TMS): 2,17 m (4H), 3,07 s (CH₃), 3,45 m (4H)

### Beispiel 12

In einem Rundhalskolben mit Rückflusskühler werden 5,77 g (0,0505 mol) 1,4-Dimethylpiperazin in 70 ml trockenen Hexan vorgelegt. Unter ständigem Rühren und Kühlen mit Hilfe eines Eisbades werden innerhalb von 20 Minuten 16,56 g (0,1009 mol) Methyltriflat zugegeben. Dann wird das Eisbad durch ein Ölbad ersetzt und die Reaktionsmischung wird 15 min unter Rückfluss gekocht (Ölbad-Temperatur 70-75°C). Nach dem Abkühlen auf Raumtemperatur wird der weiße Niederschlag abfiltriert, zweimal mit 10 ml Hexan gewaschen und bei 80°C im Vakuum bei 30-100 Pa drei Stunden getrocknet: es werden 20,68 g (Ausbeute: 92,7%) 1,1,4,4-Tetramethylpiperazinium-di-(trifluormethansulfonat) als weißer. Feststoff erhalten.

NMR ¹⁹F, ppm (Lösungsmittel: (CD₃)₂SO₂, interner Standard CCl₃F): -77,40 s (CF₃SO₃⁻)
NMR ¹H, ppm (Lösungsmittel: (CD₃)₂SO₂, interner Standard TMS): 3,30 s (4CH₃), 3,82 s (4CH₂)

Alle ¹⁹F und ¹H NMR Spektren wurden auf einem Brucker WP 80 SY Spektrometer (80,1 MHz für ¹H und 75,4 MHz für ¹⁹F) aufgenommen.

## Patentansprüche

1. Verfahren zur Herstellung von Perfluoralkansulfansäurealkylestern
**dadurch gekennzeichnet, dass** Perfluoralkansulfonsäure direkt mit Dialkylcarbonat,
wobei ein wasser- oder alkohol-verbrauchenden Reagenz, insbesondere ein Carbonsäurederivat, dessen organischer Rest gegenüber Perfluoralkansulfonsäure stabil ist, anwesend sein kann,
zu Perfluoralkansulfonsäurealkylester oder zu Perfluoralkansulfonsäurealkylester und Carbonsäureester umgesetzt wird.

2. Verfahren nach Anspruch 1,
**dadurch gekennzeichnet, dass** Perfluoralkansulfonsäure direkt mit Dialkylcarbonat und in Gegenwart eines wasser- oder alkoholverbrauchenden Reagenz, insbesondere in Gegenwart eines Carbonsäurederivates, dessen organischer Rest gegenüber Perfluoralkansulfonsäure stabil ist,
zu Perfluoralkansulfonsäurealkylester oder zu Perfluoralkansulfonsäurealkylester und Carbonsäureester umgesetzt wird.

3. Verfahren nach Anspruch 1 oder 2,
**dadurch gekennzeichnet, dass** das Dialkylcarbonat ausgewählt ist aus der Gruppe Dimethylcarbonat, Diethylcarbonat, Dipropylcarbonat, Dibutylcarbonat, Methylethylcarbonat oder eine Mischung dieser Dialkylcarbonate.

4. Verfahren nach mindestens einem der vorhergehenden Ansprüche,
**dadurch gekennzeichnet, dass** das Carbonsäurederivat ausgewählt ist aus Carbonsäurehalogenid, Carbonsäureanhydrid oder gemischtes Carbonsäure-Sulfonsäureanhydrid.

5. Verfahren nach Anspruch 4,
**dadurch gekennzeichnet, dass** das Carbonsäurehalogenid ausgewählt ist aus Benzoylchlorid, p-Nitrobenzoylchlorid, 2,6-Difluorbenzoylchlorid, Pentafluorbenzoylchlorid, 2-Chlorbenzoylchlorid, 3-Chlorbenzoylchlorid, 4-Chlorbenzoylchlorid, 2-Brombenzoylchlorid, 3-Brombenzoylchlorid, 4-Brombenzoylchlorid, 2,3-Dichlorbenzoylchlorid, 2,4-Dichlorbenzoylchlorid, 2,6-Dichlorbenzoylchlorid, 3,4-Dichlorbenzoylchlorid, 3,5-Dichlorbenzoylchlorid, Trichloracetylchlorid.

6. Verfahren nach Anspruch 4,
**dadurch gekennzeichnet, dass** das Carbonsäureanhydrid ausgewählt ist aus Benzoesäureanhydrid, 2,2'-Dichlorbenzoesäureanhydrid, 3,3'-Dichlorbenzoesäureanhydrid, 4,4'-Dichlorbenzoesäureanhydrid, 2,2',3,3'-Tetrachlorbenzoesäureanhydrid, 2,2',4,4'-Tetrachlorbenzoesäureanhydrid, 2,2',6,6'-Tetrachlorbenzoesäureanhydrid, 3,3',4,4'-Tetrachlorbenzoesäureanhydrid, 3,3',5,5'-Tetrachlorbenzoesäureanhydrid, 2-Brombenzoesäureanhydrid, 3-Brombenzoesäureanhydrid, 4-Brombenzoesäureanhydrid, 2,2',6,6'-Tetrafluorbenzoesäureanhydrid.

7. Verfahren nach mindestens einem der vorhergehenden Ansprüche,
**dadurch gekennzeichnet, dass** es bei einer Temperatur zwischen Raumtemperatur und 150°C, insbesondere zwischen 50 und 110°C, durchgeführt wird.

8. Verfahren nach mindestens einem der vorhergehenden Ansprüche,
**dadurch gekennzeichnet, dass** die Reaktionszeit zwischen 1 und 10 Stunden, insbesondere zwischen 2 und 5 Stunden, liegt.

9. Verfahren nach mindestens einem der vorhergehenden Ansprüche,
**dadurch gekennzeichnet, dass** anschließend der Perfluoralkansulfonsäurealkylester mit einer Verbindung gemäß der Formel
XR¹R²R³
umgesetzt wird, wobei
X P oder N
R¹, R², R³ gleich oder verschieden sind, gegebenenfalls durch eine Einfach- oder Doppelbildung direkt miteinander verbunden sind und jeweils einzeln oder gemeinsam die Bedeutung
- eines Wasserstoffs,
- eines Alkylrestes mit 1 bis 16 C-Atomen, der teilweise oder vollständig mit weiteren Gruppen, vorzugsweise F, Cl, N(CₙF₍₂ₙ₊₁₋ₓ₎Hₓ)₂, O(CₙF₍₂ₙ₊₁₋ₓ₎Hₓ), SO₂(CₙF₍₂ₙ₊₁₋ₓ₎Hₓ), CₙF₍₂ₙ₊₁₋ₓ₎Hₓ mit 1≤n≤6 und 0≤x≤2n+1, substituiert sein kann,
- eines Alkylarylrestes, dessen Alkylengruppe 1 bis 16 C-Atome besitzt und der teilweise mit weiteren Gruppen, vorzugsweise F, Cl, Br, NO₂, CN, Alkyl, Aryl oder heterocyclischem Aryl, substituiert sein kann,
- eines Arylrestes, der teilweise mit weiteren Gruppen, vorzugsweise F, Cl, Br, NO₂, CN, Alkyl, Aryl oder heterocyclischem Aryl, substituiert sein kann, oder
- eines aromatischen heterocyclischen Restes, der teilweise mit weiteren Gruppen, vorzugsweise F, Cl, Br, NO₂, CN, Alkyl, Aryl oder heterocyclischem Aryl, substituiert sein kann,
besitzen,
wobei ein, zwei oder drei CH₂-Gruppen eines Alkylrestes durch gleiche oder verschiedene Heteroatome, vorzugsweise O, NH oder N(Alkyl) mit 1 bis 6 C-Atomen, ersetzt sein können
und wobei nicht alle drei R gleichzeitig perfluoriert oder perchloriert sein können,
bedeutet.

## Claims

1. Process for the preparation of alkyl perfluoroalkanesulfonates,
**characterised in that** perfluoroalkanesulfonic acid is reacted directly with dialkyl carbonate,
where a water- or alcohol-consuming reagent, in particular a carboxylic acid derivative whose organic radical is stable to perfluoroalkanesulfonic acid, can be present,
to give alkyl perfluoroalkanesulfonates or to give alkyl perfluoroalkanesulfonates and carboxylic acid esters.

2. Process according to Claim 1,
**characterised in that** perfluoroalkanesulfonic acid is reacted directly with dialkyl carbonate in the presence of a water- or alcohol-consuming reagent, in particular in the presence of a carboxylic acid derivative whose organic radical is stable to perfluoroalkanesulfonic acid,
to give alkyl perfluoroalkanesulfonates or to give alkyl perfluoroalkanesulfonates and carboxylic acid esters.

3. Process according to Claim 1 or 2,
**characterised in that** the dialkyl carbonate is selected from the group dimethyl carbonate, diethyl carbonate, dipropyl carbonate, dibutyl carbonate, methyl ethyl carbonate or a mixture of these dialkyl carbonates.

4. Process according to at least one of the preceding claims,
**characterised in that** the carboxylic acid derivative is selected from carboxylic acid halide, carboxylic anhydride and mixed carboxylic/sulfonic anhydride.

5. Process according to Claim 4,
**characterised in that** the carboxylic acid halide is selected from benzoyl chloride, p-nitrobenzoyl chloride, 2,6-difluorobenzoyl chloride, pentafluorobenzoyl chloride, 2-chlorobenzoyl chloride, 3-chlorobenzoyl chloride, 4-chlorobenzoyl chloride, 2-bromobenzoyl chloride, 3-bromobenzoyl chloride, 4-bromobenzoyl chloride, 2,3-dichlorobenzoyl chloride, 2,4-dichlorobenzoyl chloride, 2,6-dichlorobenzoyl chloride, 3,4-dichlorobenzoyl chloride, 3,5-dichlorobenzoyl chloride, trichloroacetyl chloride.

6. Process according to Claim 4,
**characterised in that** the carboxylic anhydride is selected from benzoic anhydride, 2,2'-dichlorobenzoic anhydride, 3,3'-dichlorobenzoic anhydride, 4,4'-dichlorobenzoic anhydride, 2,2',3,3'-tetrachlorobenzoic anhydride, 2,2',4,4'-tetrachlorobenzoic anhydride, 2,2',6,6'-tetrachlorobenzoic anhydride, 3,3',4,4'-tetrachlorobenzoic anhydride, 3,3',5,5'-tetrachlorobenzoic anhydride, 2-bromobenzoic anhydride, 3-bromobenzoic anhydride, 4-bromobenzoic anhydride, 2,2',6,6'-tetrafluorobenzoic anhydride.

7. Process according to at least one of the preceding claims,
**characterised in that** it is carried out at a temperature between room temperature and 150°C, in particular between 50 and 110°C.

8. Process according to at least one of the preceding claims,
**characterised in that** the reaction time is between 1 and 10 hours, in particular between 2 and 5 hours.

9. Process according to at least one of the preceding claims,
**characterised in that** the alkyl perfluoroalkanesulfonate is subsequently reacted with a compound of the formula
XR¹R²R³
where
X denotes P or N,
R¹, R², R³ are identical or different, are optionally bonded directly to one another by a single or double bond and each, individually or together, have the meaning
- of a hydrogen,
- of an alkyl radical having 1 to 16 C atoms, which may be partially or fully substituted by further groups, preferably F, Cl, N(CₙF₍₂ₙ₊₁₋ₓ₎Hₓ)₂, O(CₙF₍₂ₙ₊₁₋ₓ₎Hₓ), SO₂(CₙF₍₂ₙ₊₁₋ₓ₎Hₓ), CₙF₍₂ₙ₊₁₋ₓ₎Hₓ where 1≤n≤6 and 0≤x≤2n+1,
- of an alkylaryl radical whose alkylene group has 1 to 16 C atoms and which may be partially substituted by further groups, preferably F, Cl, Br, NO₂, CN, alkyl, aryl or heterocyclic aryl,
- of an aryl radical, which may be partially substituted by further groups, preferably F, Cl, Br, NO₂, CN, alkyl, aryl or heterocyclic aryl, or
- of an aromatic heterocyclic radical, which may be partially substituted by further groups, preferably F, Cl, Br, NO₂, CN, alkyl, aryl or heterocyclic aryl,
where one, two or three CH₂ groups in an alkyl radical may have been replaced by identical or different heteroatoms, preferably O, NH or N(alkyl) having 1 to 6 C atoms, and where all three R cannot simultaneously be perfluorinated or perchlorinated.

## Revendications

1. Procédé pour la préparation de perfluoroalkanesulfonates d'alkyle,
**caractérisé en ce que** de l'acide perfluoroalkanesulfonique est amené à réagir directement avec du carbonate de dialkyle,
dans lequel un réactif consommant de l'eau ou de l'alcool, en particulier un dérivé d'acide carboxylique dont un radical organique est stable vis-à-vis de l'acide perfluoroalkanesulfonique, peut être présent,
pour obtenir des perfluoroalkanesulfonates d'alkyle ou pour obtenir des perfluoroalkanesulfonates d'alkyle et des esters d'acide carboxylique.

2. Procédé selon la revendication 1,
**caractérisé en ce que** de l'acide perfluoroalkanesulfonique est amené à réagir directement avec du carbonate de dialkyle en présence d'un réactif consommant de l'eau ou de l'alcool, en particulier en présence d'un dérivé d'acide carboxylique dont un radical organique est stable vis-à-vis de l'acide perfluoroalkanesulfonique,
pour obtenir des perfluoroalkanesulfonates d'alkyle ou pour obtenir des perfluoroalkanesulfonates d'alkyle et des esters d'acide carboxylique.

3. Procédé selon la revendication 1 ou 2,
**caractérisé en ce que** le carbonate de dialkyle est choisi parmi le groupe comprenant carbonate de diméthyle, carbonate de diéthyle, carbonate de dipropyle, carbonate de dibutyle, carbonate de méthyle éthyle ou un mélange de ces carbonates de dialkyle.

4. Procédé selon au moins l'une des revendications précédentes,
**caractérisé en ce que** le dérivé d'acide carboxylique est choisi parmi halogénure d'acide carboxylique, anhydride carboxylique et anhydride carboxylique/sulfonique mélangé.

5. Procédé selon la revendication 4,
**caractérisé en ce que** l'halogénure d'acide carboxylique est choisi parmi chlorure de benzoyle, chlorure de p-nitrobenzoyle, chlorure de 2,6-difluorobenzoyle, chlorure de pentafluorobenzoyle, chlorure de 2-chlorobenzoyle, chlorure de 3-chlorobenzoyle, chlorure de 4-chlorobenzoyle, chlorure de 2-bromobenzoyle, chlorure de 3-bromobenzoyle, chlorure de 4-bromobenzoyle, chlorure de 2,3-dichlorobenzoyle, chlorure de 2,4-dichlorobenzoyle, chlorure de 2,6-dichlorobenzoyle, chlorure de 3,4-dichlorobenzoyle, chlorure de 3,5-dichlorobenzoyle, chlorure de trichloroacétyle.

6. Procédé selon la revendication 4,
**caractérisé en ce que** l'anhydride carboxylique est choisi parmi anhydride benzoïque, anhydride 2,2'-dichlorobenzoïque, anhydride 3,3'-dichlorobenzoïque, anhydride 4,4'-dichlorobenzoïque, anhydride 2,2',3,3'-tétrachlorobenzoïque, anhydride 2,2',4,4'-tétrachlorobenzoïque, anhydride 2,2',6,6'-tétrachlorobenzoïque, anhydride 3,3',4,4'-tétrachlorobenzoïque, anhydride 3,3',5,5'-tétrachlorobenzoïque, anhydride 2-bromobenzoïque , anhydride 3-bromobenzoïque, anhydride 4-bromobenzoïque, anhydride 2,2',6,6'-tétrafluorobenzoïque.

7. Procédé selon au moins l'une des revendications précédentes,
**caractérisé en ce qu'**il est mis en oeuvre à une température entre la température ambiante et 150°C, en particulier entre 50 et 110°C.

8. Procédé selon au moins l'une des revendications précédentes,
**caractérisé en ce que** le temps de réaction est entre 1 et 10 heures, en particulier entre 2 et 5 heures.

9. Procédé selon au moins l'une des revendications précédentes,
**caractérisé en ce que** le perfluoroalkanesulfonate d'alkyle est ensuite amené à réagir avec un composé de la formule
XR¹R²R³
dans laquelle
X représente P ou N,
R¹, R², R³ sont identiques ou différents, sont en option liés directement les uns aux autres au moyen d'une liaison simple ou double et chacun, individuellement ou en association, présente la signification
- d'un hydrogène,
- d'un radical alkyle comportant de 1 à 16 atomes de C, qui peut être partiellement ou complètement substitué par d'autres groupes, de préférence F, Cl, N(CₙF₍₂ₙ₊₁₋ₓ₎Hₓ)₂, O(CₙF₍₂ₙ₊₁₋ₓ₎Hₓ), SO₂(CₙF₍₂ₙ₊₁₋ₓ₎Hₓ), CₙF(₂ₙ₊₁₋ₓ₎Hₓ où 1 ≤ n ≤ 6 et 0 ≤ x ≤ 2n+1,
- d'un radical alkylaryle dont un groupe alkylène comporte de 1 à 16 atomes de C et qui peut être partiellement substitué par d'autres groupes, de préférence F, CI, Br, NO₂, CN, alkyle, aryle ou aryle hétérocyclique,
- d'un radical aryle, qui peut être partiellement substitué par d'autres groupes, de préférence F, CI, Br, NO₂, CN, alkyle, aryle ou aryle hétérocyclique, ou
- d'un radical hétérocyclique aromatique qui peut être partiellement substitué par d'autres groupes, de préférence F, CI, Br, NO₂, CN, alkyle, aryle ou aryle hétérocyclique,
où un, deux ou trois groupes CH₂ dans un radical alkyle peuvent avoir été remplacés par des hétéroatomes identiques ou différents, de préférence O, NH ou N(alkyle) comportant de 1 à 6 atomes de C,
et où les trois R ne peuvent pas être tous simultanément perfluorés ou perchlorés.
